# EUROPEAN PATENT SPECIFICATION

(11) **EP 3 501 518 B1**
(45) Date of publication and mention of the grant of the patent: **14.05.2025**
(21) Application number: 17843502.0
(22) Date of filing: 21.08.2017
(51) Int. Cl.: A61K 9/70, A61K 47/12, A61K 31/27, A61K 31/202, A61P 25/28

(54) **TRANSDERMAL PATCH OF RIVASTIGMINE**
TRANSDERMALES PFLASTER MIT RIVASTIGMIN
PATCH TRANSDERMIQUE DE RIVASTIGMINE

(30) Priority: 22.08.2016 JP 2016161730
(43) Date of publication of application: 26.06.2019
(73) Proprietor: Kyukyu Pharmaceutical Co., Ltd., Tokyo 103-0023 (JP)
(72) Inventor: FUTATSUYA, Akihiro, Imizu-shi Toyama 939-0351 (JP)
(74) Representative: Wächtershäuser & Hartz Patentanwaltspartnerschaft mbB
(86) International application number: PCT/JP2017/029697
(87) International publication number: WO 2018/038022

(56) References cited:
- WO-A1-2007/099966
- WO-A1-2015/087926
- WO-A2-2012/087047
- JP-A- 2011 020 997
- JP-A- 2014 508 728
- JP-A- S59 216 808
- RAGICEVIC, NINA, MAIBACH, HOWARD I.: "Percutaneous Penetration Enhancers Chemical Methods in Percutaneous Penetration Enhancement : Modification of the Stratum Corneum", part 9 2015, SPRINGER-VERLAG, Berlin Heidelberg, ISBN: 978-3-662-47039-8, article R. JAYACHANDRA BABU, LI CHEN, NARAYAN KANIKKANNAN: "Fatty Alcohols, Fatty Acids, and Fatty Acid Esters as Penetration Enhancers", pages: 133 - 144, XP002798060

## Description

### Field of the Invention

The present invention relates to a non-aqueous patch, comprising:a support;a drug-containing pressure-sensitive adhesive layer; and a release liner,wherein the drug-containing pressure-sensitive adhesive layer comprises:rivastigmine; and a rubber-based pressure-sensitive adhesive, characterized in that the drug-containing pressure-sensitive adhesive layer comprises:from 0.3 % by mass to 1 % by mass of a saturated fatty acid having 20to 24 carbon atoms, and the rubber-based pressure-sensitive adhesive comprises a pressure-sensitive adhesive containing a styrene-isoprene-styrene block copolymer and polyisobutylene.

### Background of the Invention

Rivastigmine is an acetylcholinesterase inhibitor, and is used as a therapeutic agent for Alzheimer-type dementia. Rivastigmine is administered in a form of a patch.

As a rivastigmine-containing patch, there have been reported a transdermal therapeutic system formed of (backing layer)/(drug reservoir layer)/(silicon polymer-containing adhesive layer) (Patent Literature 1), and a transdermal therapeutic system formed of (cover layer) / (active substance layer) / (drug release controlling membrane) / (adhesive layer) / (pull-off layer) (Patent Literature 2) . However, each of those preparations has a complex preparation structure and also entails a complex manufacturing process, and hence has problems of entailing a high manufacturing cost and having a poor attachment property owing to an increased thickness of the preparation.

Meanwhile, as a so-called matrix-type rivastigmine patch, which is formed of (support)/(drug-containing pressure-sensitive adhesive layer)/(liner), the following ones are known: (1) a rivastigmine patch using an acrylic pressure-sensitive adhesive, in which oxidative decomposition of rivastigmine is suppressed by blending an organic acid into the drug-containing layer without using an antioxidant (Patent Literature 3); (2) a rivastigmine patch of a styrene-isoprene-styrene block copolymer (SIS)-based base, in which skin irritation is reduced by adding a large amount of a non-volatile hydrocarbon oil and adding no tackifier (Patent Literature 4); and (3) a patch obtained by adding rivastigmine to a pressure-sensitive adhesive that is formed of SIS, a tackifier resin, and a plasticizer and does not contain an organic acid or an organic acid salt (Patent Literature 5).

### Prior Art

### Patent Literature

[PTL 1] JP 2009-517468 A
[PTL 2] JP 2014-503523 A
[PTL 3] JP 2016-069287 A
[PTL 4] JP 2013-187451 A1
[PTL 5] JP 2014-508728 A
R. Jayachandra Babu, et al. , Percutaneous Penetration Enhancers Chemical Methods in Percutaneous Penetration Enhancement : Modification of the Stratum Corneum, Berlin Heidelberg, Springer-Verlag, (2015), pages 133 - 144, 9, ISBN 978-3-662-47039-8 , discloses fatty Alcohols, fatty Acids, and fatty acid esters as penetration enhancers.

### Summary of the Invention

### Problem to be solved by the invention

However, the results of Examples of Patent Literature 5 reveal that the skin permeability of rivastigmine significantly varies depending on the drug concentration in the preparation, the thickness of the drug layer, and the kind of the tackifier resin. In particular, a change in thickness of the drug layer may affect affixing feeling by causing changes in preparation weight and preparation stiffness.

Therefore, an object of the present invention is to provide means capable of enhancing the skin permeability of rivastigmine without adversely affecting the pressure-sensitive adhesive property of a patch.

### Means for solving the problem

In view of the foregoing, the inventor of the present invention made various investigations on a component for enhancing the skin permeability of rivastigmine without lowering the pressure-sensitive adhesive property of a matrix-type patch, and as a result, found that a non-aqueous patch excellent in pressure-sensitive adhesive property and having a high skin permeation rate of rivastigmine was obtained by adding a long-chain fatty acid in an amount as small as 0.3 % by mass to 1 % by mass to a rubber-based base containing a styrene-isoprene-styrene block copolymer and polyisobutylene.

The present invention relates to the subject matter of claims 1 to 3.

### Advantageous Effects of Invention

The rivastigmine-containing non-aqueous patch of the present invention is a patch having a high skin permeation rate of rivastigmine and an improved pressure-sensitive adhesive force, thereby being capable of maintaining drug efficacy for a long period of time. In addition, because the addition amount of the saturated fatty acid having 20 to 24 carbon atoms is small, the thickness of the drug layer is thin and does not cause changes in preparation weight and preparation stiffness, and hence affixing feeling is also satisfactory.

### Brief Description of Drawings

FIG. 1 is a conceptual diagram of a pressure-sensitive adhesive force test.
FIG. 2 is a graph for showing the skin permeation rate of rivastigmine of each of Examples and Comparative Examples.
FIG. 3 is a graph for showing the pressure-sensitive adhesive force of each of Examples and Comparative Examples.

### Description of Embodiments

A patch of the present invention comprises a drug-containing pressure-sensitive adhesive layer that contains: rivastigmine; 0.3 % by mass to 1 % by mass of a saturated fatty acid having 20 to 24 carbon atoms; and a rubber-based pressure-sensitive adhesive containing a styrene-isoprene-styrene block copolymer and polyisobutylene.

The drug-containing pressure-sensitive adhesive layer contains rivastigmine as an active substance. From the viewpoints of obtaining an effective blood concentration and preventing skin irritation, the content of rivastigmine in the drug-containing pressure-sensitive adhesive layer is preferably from 10 % by mass to 25 % by mass, more preferably from 10 % by mass to 20 % by mass.

The drug-containing pressure-sensitive adhesive layer of the patch of the present invention contains 0.3 % by mass to 1 % by mass of the saturated fatty acid having 20 to 24 carbon atoms. Examples of the fatty acid include arachidic acid, behenic acid, and lignoceric acid. Of those, behenic acid is more preferred. When 0.3 % by mass or more of the fatty acid is contained, the skin permeation rate of rivastigmine is enhanced, and besides, a pressure-sensitive adhesive force is improved. When the content of the fatty acid is less than 0.3 % by mass, the pressure-sensitive adhesive force is lowered, and besides, a sufficient enhancement of the skin permeation rate of rivastigmine is not obtained. From the viewpoints of enhancing the skin permeation rate and improving the pressure-sensitive adhesive force, and from the viewpoint of preventing softening of the pressure-sensitive adhesive, the content of the fatty acid is from 0.3 % by mass to 1 % by mass, preferably from 0.3 % by mass to 0.9 % by mass, more preferably from 0.3 % by mass to 0.8 % by mass, even more preferably from 0.3 % by mass to 0.7 % by mass.

A base of the drug-containing pressure-sensitive adhesive layer is a rubber-based pressure-sensitive adhesive comprising a styrene-isoprene-styrene block copolymer (SIS) and polyisobutylene (PIB) in combination for obtaining satisfactory affixing feeling.

Examples of the styrene-isoprene-styrene block copolymer include: D1111, D1113, D1119, D1161, and D1165 manufactured by Kraton Corporation; SIS5002, SIS5250, and SIS5505 manufactured by JSR Corporation; and Quintac 3620, Quintac 3433, Quintac 3520, Quintac 3450, and Quintac 3280 manufactured by Zeon Corporation.

From the viewpoints of the shape-retaining property of the pressure-sensitive adhesive layer and the prevention of peeling, the content of the styrene-isoprene-styrene block copolymer in the drug-containing pressure-sensitive adhesive layer is preferably from 25 % by mass to 60 % by mass, more preferably from 30 % by mass to 45 % by mass.

In addition, the styrene-isoprene-styrene block copolymer allows the hardness of the pressure-sensitive adhesive to be adjusted through combined use of grades different from each other in polymerization ratio between styrene and isoprene.

From the viewpoints of obtaining satisfactory affixing feeling and preventing the pressure-sensitive adhesive from sticking out during storage, the content of polyisobutylene in the drug-containing pressure-sensitive adhesive layer is preferably from 0.5 % by mass to 5 % by mass, more preferably from 0.5 % by mass to 3 % by mass.

It is preferred to add 1 part by mass or more, more preferably 2 parts by mass or more of polybutene with respect to 1 part by mass of polyisobutylene.

As described later, when polyisobutylene is dissolved in polybutene, even in a solvent method using ethyl acetate as a solvent, the dissolution time of a coating solution can be shortened, and hence a heat load on the drug can be reduced.

The drug-containing pressure-sensitive adhesive layer of the patch of the present invention may further contain a tackifier, a plasticizer, a softener, a transdermal absorption enhancer, an antioxidant or an excipient.

Examples of the tackifier include hydrogenated rosin glycerol ester, an alicyclic saturated hydrocarbon resin, an aliphatic saturated hydrocarbon resin, ester gum, rosin, a phenol resin, a terpene resin, and a petroleum resin. Of those, hydrogenated rosin glycerol ester is preferred. The content of the tackifier in the drug-containing pressure-sensitive adhesive layer is preferably from 30 % by mass to 50 % by mass, more preferably from 35 % by mass to 45 % by mass.

Examples of the plasticizer and the softener include polybutene, liquid paraffin, light liquid paraffin, a medium-chain fatty acid triglyceride, triacetin, squalane, squalene, and castor oil. The content of each of the plasticizer and the softener in the drug-containing pressure-sensitive adhesive layer is preferably from 1 % by mass to 10 % by mass, more preferably from 1 % by mass to 6 % by mass.

Examples of the transdermal absorption enhancer include: fatty acid esters, such as isopropyl myristate, butyl myristate, isopropyl palmitate, isopropyl isostearate, diethyl sebacate, diisopropyl sebacate, and diethyl adipate; polyhydric alcohol fatty acid esters, such as propylene glycol monocaprylate and propylene glycol dicaprylate; and aliphatic alcohols, such as myristyl alcohol and lauryl alcohol.

Examples of the antioxidant include dibutylhydroxytoluene, butylhydroxyanisole, benzotriazole, mercaptobenzimidazole, sodium edetate, a sulfite, and a bisulfite. Of those, benzotriazole or mercaptobenzimidazole is preferred from the viewpoints of the stabilization of rivastigmine and a suppressing effect on the decoloring of a preparation. Examples of the excipient include kaolin, talc, bentonite, light anhydrous silicic acid, anhydrous silicic acid, aluminum silicate, titanium oxide, zinc oxide, starch acrylate, methyl cellulose, ethyl cellulose, hydroxypropyl cellulose, sodium carboxymethyl cellulose, hydroxypropyl methylcellulose, povidone, and sodium polyacrylate.

As a support of the patch of the present invention, there are given: a single plastic film of polyethylene terephthalate, polyethylene, polypropylene, nylon, ethylene vinyl alcohol, or the like; a support obtained by subjecting the surface of such plastic film to corona discharge treatment so as to facilitate the attachment of the pressure-sensitive adhesive; and a support obtained by bonding a nonwoven fabric of polyethylene terephthalate, polyethylene or polypropylene to such plastic film so as to facilitate the attachment of the pressure-sensitive adhesive.

Of those, a support obtained by bonding a polyethylene terephthalate nonwoven fabric to a polyethylene terephthalate film is preferred because of, for example, being less liable to adsorb the drug, having high barrier properties, and having good attachability and visibility of the pressure-sensitive adhesive.

As a release liner of the patch of the present invention, there is given a release liner obtained by subjecting a plastic film of polyethylene terephthalate, polyethylene or polypropylene to silicone treatment or fluorine treatment.

As a manufacturing method for the patch of the present invention, there are given: a solvent method, which involves dissolving/mixing formulation components in an organic solvent, such as toluene, hexane, or ethyl acetate, spreading the resultant, and then evaporating the organic solvent; and a hot-melt method, which involves hot-melting a base component, then adding the active substance and spreading the resultant.

Of those, a solvent method is preferred from the viewpoint that the heat load on the drug can be reduced.

Further, the organic solvent to be used for the solvent method is preferably ethyl acetate from the viewpoint of the toxicity of the solvent as shown in the Guideline for Residual Solvents in Pharmaceuticals (PMSB/ELD Notification No. 307).

### Examples

Next, the present invention is described in more detail by way of Examples.

### (1) Production of Patch

Preparations of formulations shown in Table 1 were produced by the following method.

### (Preparation of Coating Solution)

### Examples 1 to 3, and Comparative Examples 1 and 2

Polyisobutylene (PIB, viscosity-average molecular weight: 50,000) was added to polybutene (number-average molecular weight: 720), and was mixed therewith by stirring (80°C) to give a PIB-dissolved material.

Rivastigmine, a styrene-isoprene-styrene block copolymer (SIS), hydrogenated rosin glycerol ester (HRGE), behenic acid, and the PIB-dissolved material were added to ethyl acetate, and were dissolved therein by stirring (60°C) to provide a coating solution. The time taken for dissolution was within 2 hours.

### Example 4

Rivastigmine, a styrene-isoprene-styrene block copolymer (SIS), hydrogenated rosin glycerol ester (HRGE), behenic acid, PIB, and polybutene were added to ethyl acetate, and were dissolved therein by stirring (60°C) to provide a coating solution. Undissolved PIB remained at a stirring time of 2 hours, and the time taken for dissolution was 4 hours.

For the styrene-isoprene-styrene block copolymer (SIS), one having a styrene ratio of 22% (SIS (1)) was used alone or in combination with one having a styrene ratio of 16% (SIS (2)).

### (Spread and Drying, and Cutting)

The coating solution was spread to the silicone-treated surface of silicone-treated polyethylene terephthalate, and the coating solution was dried by blowing hot air at 65°C to provide a pressure-sensitive adhesive layer. A support was laminated on the pressure-sensitive adhesive layer, and then the laminate was cut to 22.6 mm×22.6 mm, R=3.5 mm to provide a patch. Separately, the laminate was cut to a size of 10 mm wide by 22.6 mm long to give a pressure-sensitive adhesive force test sample.

For the support, one obtained by bonding a nonwoven fabric of polyethylene terephthalate to a film of polyethylene terephthalate was used. The pressure-sensitive adhesive layer was applied to the nonwoven fabric surface.

The spreading amount was adjusted so that the amount of rivastigmine in one patch was 9 mg.

### (2) Skin Permeation Test

An abdominal skin excised from a 7-weeks-old male hairless mouse was mounted on a horizontal diffusion cell (Chem. Pharm. Bull. 37(5), 1404-1406, 1989), and a predetermined preparation sample was applied to a stratum corneum side.

Meanwhile, 2.5 mL of an isotonic phosphate buffer (pH 7.4) was applied as a receiver fluid to a receiver side (dermis side).

During the test, the receiver fluid was kept at 32°C and stirred using a magnetic stirrer. At predetermined time, 1 mL of the receiver fluid was collected as a sample solution. Immediately after the collection of the sample solution, 1 mL of a fresh isotonic phosphate buffer was added.

The rivastigmine in the sample solution was quantified by high performance liquid chromatography to determine the cumulative permeation amount (µg/cm²) of rivastigmine.

Further, the permeation rate (µg/cm²/h) of rivastigmine was determined by a least-squares method from a relationship between the cumulative permeation amount at 4 hours, 6 hours, and 8 hours after the start of the test and time.

### (3) Pressure-sensitive Adhesive Force Test

Measurement was performed with a tensile tester (Texture Analyzer TA.XT plus, EKO Instruments) by the following procedure.

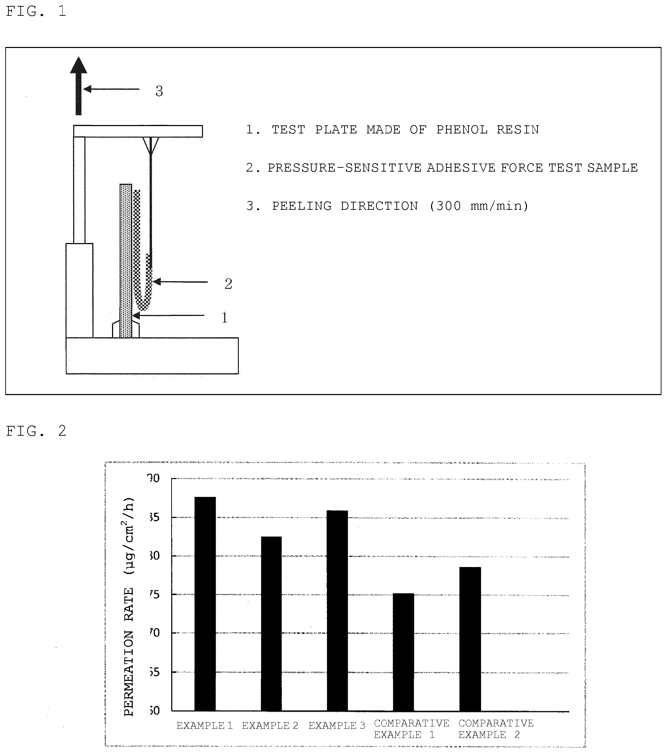

The pressure-sensitive adhesive force test sample (width: 10 mm, length: 22.6 mm) was attached with one end aligned to a test plate made of a phenol resin, which had been kept in an incubator at 37°C. A rubber roller having a mass of 850 g was allowed to pass across the attached pressure-sensitive adhesive force test sample twice, and then the sample was placed in an incubator at 37°C and left at rest therein for 30 minutes. Next, the sample was left at rest at room temperature for 15 minutes, and then measured for its pressure-sensitive adhesive force (see FIG. 1).

The measurement was performed as follows: the pressure-sensitive adhesive force test sample was set in the tensile tester with one side thereof having a width of 10 mm folded back at 180°, and was continuously peeled off at a rate of 300 mm/min, and the average value of a load in this case was determined and defined as a pressure-sensitive adhesive force (g/10 mm).

### (4) Test Results

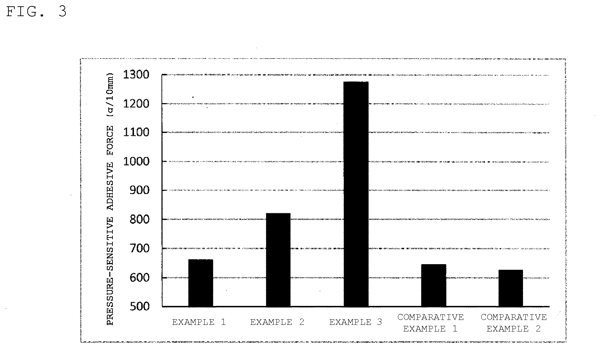

In Table 2, the results of the skin permeation test and the pressure-sensitive adhesive force test are shown. In addition, in FIG. 2 and FIG. 3, the results are graphically shown.

In Comparative Example 2, in which 0.1 % by mass of behenic acid was added to Comparative Example 1, the skin permeation rate and the pressure-sensitive adhesive force were enhanced only slightly. However, when 0.3 % by mass or more of behenic acid was added, a clear enhancement was found.

In addition, as shown in the section for the preparation of the coating solution, when PIB was dissolved in polybutene and then added to ethyl acetate, the dissolution time of the coating solution was able to be reduced by more than half.

This is because PIB is insoluble in ethyl acetate, but dissolves in ethyl acetate having dissolved therein SIS. It is considered that the dissolution of SIS in ethyl acetate lowered the polarity of the liquid to allow PIB to dissolve.

That is, it is considered that the dissolution time was able to be shortened by the time taken for the dissolution of SIS in ethyl acetate.

With this, the time taken for the step of preparing the coating solution can be shortened, that is, the heat load on the drug can be reduced. This is more useful for a drug known to have poor thermal stability, such as rivastigmine.

**Table 1**

| Component name | Example 1 | Example 2 | Example 3 | Comparative Example 1 | Comparative Example 2 | Example 4 |
|---|---|---|---|---|---|---|
| Rivastigmine | 16 | 16 | 16 | 16 | 16 | 16 |
| SIS (1) | 38.7 | 38.5 | 18.5 | 39 | 38.9 | 18.5 |
| SIS (2) | - | - | 20 | - | - | 20 |
| HRGE | 42 | 42 | 42 | 42 | 42 | 42 |
| Behenic acid | 0.3 | 0.5 | 0.5 | - | 0.1 | 0.5 |
| PIB | 1 | 1 | 1 | 1 | 1 | 1 |
| Polybutene | 2 | 2 | 2 | 2 | 2 | 2 |
| Total | 100 | 100 | 100 | 100 | 100 | 100 |

**Table 2**

| Time (h) | Example 1 | Example 2 | Example 3 | Comparative Example 1 | Comparative Example 2 |
|---|---|---|---|---|---|
| 2 | 71.0 | 54.4 | 59.2 | 43.8 | 49.8 |
| 4 | 219.4 | 181.6 | 192.9 | 161.8 | 174.1 |
| 6 | 397.8 | 347.8 | 365.0 | 310.8 | 333.3 |
| 8 | 569.9 | 511.7 | 536.4 | 462.7 | 488.5 |
| Permeation rate | 87.6 | 82.5 | 85.9 | 75.2 | 78.6 |
| Pressure-sensitive adhesive force | 661 | 820 | 1,275 | 645 | 627 |

### Preparation Examples 1 and 2

Preparations of formulations shown in Table 3 were produced by the same method as that of Examples 1 to 3. Mercaptobenzimidazole or benzotriazole dissolved in ethyl acetate.

**Table 3**

| Component name | Preparation Example 1 | Preparation Example 2 |
|---|---|---|
| Rivastigmine | 16 | 16 |
| SIS (1) | 38.4 | 38.2 |
| SIS (2) | - | - |
| HGRE | 42 | 42 |
| Behenic acid | 0.5 | 0.5 |
| PIB | 1 | 1 |
| Polybutene | 2 | 2 |
| Mercaptobenzimidazole | 0.1 | - |
| Benzotriazole | - | 0.3 |
| Total | 100 | 100 |

## Claims

1. A non-aqueous patch, comprising:
a support;
a drug-containing pressure-sensitive adhesive layer; and
a release liner,
wherein the drug-containing pressure-sensitive adhesive layer comprises:
rivastigmine; and
a rubber-based pressure-sensitive adhesive,
**characterized in that**
the drug-containing pressure-sensitive adhesive layer comprises:
from 0.3 % by mass to 1 % by mass of a saturated fatty acid having 20 to 24 carbon atoms, and
the rubber-based pressure-sensitive adhesive comprises a pressure-sensitive adhesive containing a styrene-isoprene-styrene block copolymer and polyisobutylene.

2. The non-aqueous patch according to claim 1, wherein the drug-containing pressure-sensitive adhesive layer contains 10 % by mass to 25 % by mass of the rivastigmine.

3. The non-aqueous patch according to claim 1 or 2, wherein the saturated fatty acid having 20 to 24 carbon atoms comprises behenic acid.

## Patentansprüche

1. Ein nichtwässriges Pflaster, umfassend:
eine Unterstützungsschicht (Support);
eine arzneimittelhaltige druckempfindliche Adhäsivschicht; und
eine Trennfolie,
wobei die arzneimittelhaltige druckempfindliche Adhäsivschicht umfasst:
Rivastigmin; und
ein druckempfindliches Adhäsiv auf Gummibasis,
**dadurch gekennzeichnet, dass**
die arzneimittelhaltige druckempfindliche Adhäsivschicht umfasst:
0,3 Masse-% bis 1 Masse-% einer gesättigten Fettsäure mit 20 bis 24 Kohlenstoffatomen und
das druckempfindliche Adhäsiv auf Gummibasis ein druckempfindliches Adhäsiv umfasst, das ein Styrol-Isopren-Styrol-Blockcopolymer und Polyisobutylen enthält.

2. Das nichtwässriges Pflaster nach Anspruch 1, wobei die arzneimittelhaltige druckempfindliche Adhäsivschicht 10 Masse-% bis 25 Masse-% des Rivastigmins enthält.

3. Das nichtwässriges Pflaster nach Anspruch 1 oder 2, wobei die gesättigte Fettsäure mit 20 bis 24 Kohlenstoffatomen Behensäure umfasst.

## Revendications

1. Timbre non aqueux, comprenant :
un support ;
une couche adhésive sensible à la pression et contenant un médicament ; et
une doublure détachable,
dans lequel la couche adhésive sensible à la pression et contenant un médicament comprend :
du rivastigmine ; et
un adhésif à base de caoutchouc sensible à la pression, **caractérisé en ce que**
la couche adhésive sensible à la pression et contenant un médicament comprend :
de 0,3 % en masse à 1 % en masse d'un acide gras saturé présentant de 20 à 24 atomes de carbone, et
l'adhésif sensible à la pression à base de caoutchouc comprend un adhésif sensible à la pression contenant un copolymère séquencé de styrène-isoprène-styrène et du polyisobutylène.

2. Timbre non aqueux selon la revendication 1, dans lequel la couche adhésive sensible à la pression et contenant un médicament contient de 10 % en masse à 25 % en masse de rivastigmine.

3. Timbre non aqueux selon la revendication 1 ou 2, dans lequel l'acide gras saturé présentant de 20 à 24 atomes de carbone comprend de l'acide béhénique.
